# EUROPEAN PATENT SPECIFICATION

(11) **EP 1 666 032 B1**
(45) Date of publication and mention of the grant of the patent: **08.02.2017**
(21) Application number: 04772762.3
(22) Date of filing: 03.09.2004
(51) Int. Cl.: A61K 9/70, A61K 47/26, A61K 47/32

(54) **LOWLY IRRITATIVE ADHESIVE PATCH**
WENIG REIZENDES KLEBEPFLASTER
TIMBRE ADHESIF PEU IRRITANT

(30) Priority: 22.09.2003 JP 2003330216
(43) Date of publication of application: 07.06.2006
(73) Proprietor: Hisamitsu Pharmaceutical Co., Inc., Tosu-shi, Saga 841-0017 (JP)
(72) Inventor: YOSHINAGA, Takaaki, Tosu-shi, Saga 841-0017 (JP); WAKAMATSU, Masato, Tosu-shi, Saga 841-0017 (JP); SHIRAI, Masato, Tosu-shi, Saga 841-0017 (JP); SAEKI, Masakazu, Tosu-shi, Saga 841-0017 (JP)
(74) Representative: Beckmann, Claus
(86) International application number: PCT/JP2004/012813
(87) International publication number: WO 2005/034925

(56) References cited:
- EP-A- 0 279 977
- EP-A- 1 074 251
- WO-A-02/08351
- JP-A- 7 048 554
- JP-A- 8 081 331
- JP-A- 8 143 738
- JP-A- 9 328 673
- JP-A- 60 123 417
- JP-A- 63 216 817
- JP-A- 2000 256 189
- JP-A- 2003 001 095
- JP-A- 2003 146 832
- JP-B2- 2 632 838
- US-A- 4 956 171
- US-A- 6 139 866

## Description

### Technical Field

The present invention relates to a sucrose stearic acid ester as a component in an adhesive patch for external use which is significantly inhibited from causing a rash and is less irritative to the skin.

### Background Art

Conventionally, as an adhesive patch for external use, there are plaster agents, cataplasm agents, tape agents, and the like as a pharmaceutical agent which are obtained by incorporating a drug into an adhesive polymer and directly or indirectly extending the resultant on a backing such as flexible cloth, non-woven cloth, or various plastic films. Also, a surgical tape, an adhesive plaster, a taping, and the like have been used as an adhesive patch that does not contain a drug.

These adhesive patches for external use have raised a problem of side-effects in use such as generation of stress on the skin at the time of application, tension of the skin and/or the hair at the time of peeling off, chemical actions caused by irritative components contained in the raw materials, and physiological actions such as rubor and rash caused by stuffiness with steam and perspiration due to long-term application. As a major cause therefor, in a conventional adhesive patch for external use, one can mention the occurrence of tissue destruction of the skin surface caused by generation of stress on the skin at the time of application, tension of the skin and/or the hair at the time of peeling off, or the like.

On the other hand, various attempts have been made as means for solving these problems. For example, Patent Document 1 discloses a method of opening an air-vent hole in a plaster. Also, Patent Document 2, Patent Document 3, and Patent Document 4 show blending of drugs for prevention of rash or reduction of skin irritation. Patent Document 5 discloses a method of performing a special treatment on a base so as to reduce the skin irritation. Also, Patent Document 6 shows removal of physiological factors of rash caused by stuffiness with steam, perspiration, or the like and removal of physical factors of rash caused by tension of hair at the time of peeling off or the like, by addition of a water-absorptive polymer. However, in all of the cases, there are practical problems (for example, lowering of adhesiveness caused by blending, or the like); and the production steps are complex; and the effects thereof are insufficient. Although various methods have been attempted, they are far from practical use, so that it is eventually the status-quo that ideal adhesive patches for external use without causing a rash have not appeared yet.

In the meantime, sucrose fatty acid esters are conventionally blended in a large amount in foods in order to produce effects such as improvement of tastiness, impartation of jelly strength, prevention of release of water, prevention of separation of oils and fats, prevention of denaturing, improvement of miscibility, and prevention of precipitation of crystals. Also, sucrose fatty acid esters are blended in cosmetics for use as an emulsifying agent or a foaming agent, or by further expecting an effect such as retention of moisture balance by formation of a thin film on the skin. Also, in medical drugs, sucrose fatty acid esters are used by expecting a lubrication effect in the step of producing an oral solid pharmaceutical agent. However, there have been no examples in which sucrose fatty acid esters are blended in adhesive patches for external use for the purpose of preventing a rash.

Patent Document 1: JP-B-S58-52251
Patent Document 2: JP-A-S58-4721
Patent Document 3: JP-A-S60-56911
Patent Document 4: JP-A-S60-23312
Patent Document 5: JP-B-S59-19528
Patent Document 6: Japanese Patent No. 2632838

### Disclosure of the Invention

The present invention has been made in view of the status-quo such as described above, and an object thereof is to provide a sucrose stearic acid ester as a component in an adhesive patch for external use which is significantly inhibited from causing a rash and is less irritative to the skin while sufficiently retaining the adhesiveness to the skin.

In order to solve the aforementioned problems, the present inventors have repeated eager researches by focusing attention on the generation of stress on the skin at the time of application and at the time of peeling off an adhesive patch. As a result, the present inventors have found out that the object can be achieved by blending a sucrose stearic acid ester, thereby completing the present invention.

Namely, the present invention relates to a sucrose stearic acid ester as a component in an adhesive patch for external use for use in the reduction of skin irritation and rash, wherein the adhesive patch is formed by incorporating the sucrose stearic acid ester into an adhesive base in an amount of 0.1 to 5.0 wt%.

The present invention produces conspicuous effects in that the present invention provides a sucrose stearic acid ester as a component in an ideal, lowly irritative adhesive patch for external use with the generation of rash reduced to a great extent and with the skin irritation restrained to the minimum by removing the physical actions such as the restraint of the stress onto the skin at the time of application and at the time of peeling off while sufficiently retaining the adhesiveness to the skin, which properties have been inconceivable in conventional adhesive patches for external use.

### Brief Description of the Drawing

[Fig. 1]

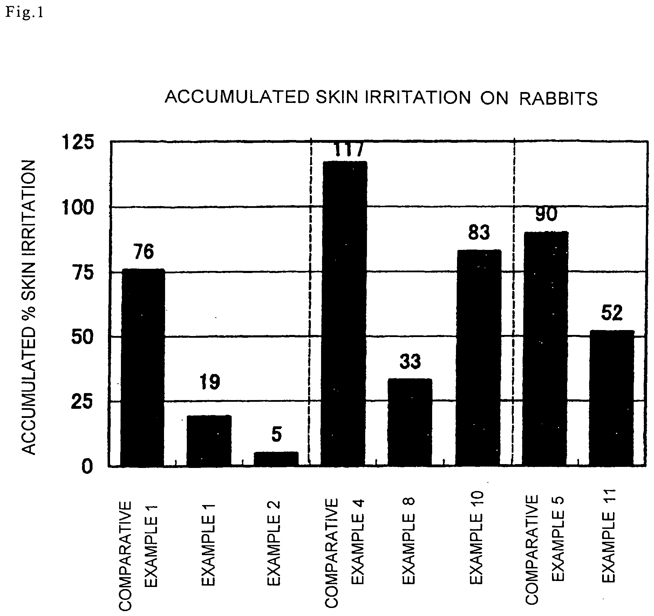

Fig. 1 shows a result of accumulated skin irritation evaluation on rabbits obtained in Test Example 3.

### Best Modes for Carrying Out the Invention

The adhesive patch used in the present invention is, for example, an adhesive patch for external use formed by directly or indirectly extending and applying an adhesive base onto a backing.

The present invention is characterized in that a sucrose stearic acid ester is incorporated into the adhesive base of an adhesive patch for external use for the purpose of reducing the generation of rash and reducing the skin irritation.

In details, by blending a sucrose stearic acid ester as an essential component so as to restrain the generation of stress onto the skin, the problem that could not be solved by blending a water-absorptive polymer or the like that was conventionally used for such a purpose, namely a side-effect such as a rash, is considerably reduced.

Generally, when the stress at the time of application or at the time of peeling off is restrained, the adhesiveness will lower, causing drop-off from the surface of the skin. Therefore, it has been hitherto considered that it is not possible to blend those that restrain the stress at the time of application and at the time of peeling off.

However, the present inventors have unexpectedly found out that a sucrose stearic acid ester can sufficiently retain the adhesiveness with a small amount of blending, producing a rash preventing effect. In other words, with a specific blending amount described later, in addition to retaining the adhesiveness more preferably, physical actions such as the generation of stress onto the skin at the time of application and at the time of peeling off are also reduced.

In an adhesive patch used in the present invention, a sucrose stearic acid ester is blended with an adhesive base.

Specific trade names may be, for example, Ryoutou Sugar Ester, Surf Hope J, Surf Hope SE PHARMA of Mitsubishi-Kagaku Foods Corporation, DK Ester of DAI-ICHI KOGYO SEIYAKU CO., LTD., and the like and are suitably selected for use in accordance with the kind of the stearic acid and the HLB.

The blending amount of the sucrose stearic acid ester constituting an essential component in the adhesive patch of the present invention is 0.1 to 5.0 wt% in the adhesive base. When the amount is too low, sufficient effect of restraining the stress cannot be expected, whereby a rash is generated.

On the other hand, when the amount is too high, although the effect of restraining the stress is sufficient, decrease in the aggregation power of the plaster and decrease in the adhesive strength are large, whereby liable to be peeled off easily.

The kind of the adhesive base to be used in the present invention is not particularly limited and may be, for example, what is known as a rubber base or a water-soluble polymer base. In an adhesive patch used in the present invention which an adhesive base is a water-soluble polymer, water, the water-soluble polymer, and the sucrose stearic acid ester are typically incorporated into the adhesive base.

The adhesive component of the adhesive base used in the present invention is not particularly limited; however, it may be, for example, a polymer substance such as silicone, styrene-isoprene-styrene, styrene-butadiene, acrylic, vinyl ether, natural rubber, polyisoprene, urethane, polyisobutylene, or acrylic acid polymer. A preferable amount of these polymer substances to be incorporated into the plaster is 10 to 90 wt%. Among these, in the case of what is known as diene rubbers such as natural rubber, polyisoprene, styrene-isoprene-styrene, and polyisobutylene, 20 to 50 wt% is especially preferable. Specifically, the effect of the adhesive patch used in the present invention is conspicuously exhibited, for example, by using a base containing a styrene-isoprene-styrene copolymer, polyisobutylene, and/or polyisoprene and blending a sucrose stearic acid ester into the base.

Also, by further incorporating polybutene into the base containing a styrene-isoprene-styrene copolymer, polyisobutylene, and/or polyisoprene, the adhesive patch used in the present invention can be used without being peeled off during the application.

Here, in the case of what is known as a water-soluble polymer such as an acrylic acid polymer, 10 to 50 wt% is especially preferable.

Into the adhesive base used in the present invention, a tackifier that is conventionally used, for example, a rosin resin [Ester Gum (Arakawa Chemical Industries,Ltd.), Hariester (Harima Chemicals, Inc.), Haritac (Harima Chemicals, Inc.)], terpene resin [YS Resin (Yasuhara Yushi), Picolite (Hercules)], petrol resin [Alcon (Arakawa Chemical Industries,Ltd.), Legaletts (Hercules), Escolets (Exxon), Wingtak (Goodyear), Quinton (Zeon)], phenolic resin, or xylene resin, may further be blended at an amount of 50 wt% or less in accordance with the needs.

However, when, for example, an acrylic base is to be used as a polymer substance of an adhesive component, the acrylic base itself may have a sufficient adhesiveness depending on the kind, so that in such a case, an tackifier need not be used. In addition to these, a plasticizer, a filler, and a stabilizer may be suitably blended. In addition, zinc oxide, titanium oxide, aluminum oxide, magnesium oxide, iron oxide, or the like may be suitably blended as metal oxide.

In the adhesive patch used in the present invention, as a drug to be incorporated into the adhesive base, any drug may be incorporated as long as it is a percutaneous absorbable agent, and there is no particular limitation.

Examples of epispastic agent and antalgic antiphlogistic agent include salicylic acid, methyl salicylate, glycol salicylate, 1-menthol, camphor (d-, 1-, dl-), menthol oil, thymol, nicotinic acid benzyl ester, capsicum extract, capsaicin, nonylic acid vanillylamide, ferbinac, butyl flufenamate, piroxicam, indomethacin, ketoprophen, planoprophen, feprazon, flurbiprofen, roxoprophen, amfenac sodium, oxaprozin, emorphazon, thiaprophen, fenbufen, planoprophen, fentiazac, diclofenac sodium, diflunisal, ibuprophen piconol, bendazac, and suprofen, as well as the ester derivatives of these, or buprenorphine hydrochloride, pentazocine, butorphanol tartrate, and others.

Examples of antifungal agent include bihonazole, clotrimazole, tioconazole, miconal, econazole, isoconazole, sulconazole, oxyconazole, cloconazole, ketoconazole, neticonazole, lanoconazole, omoconazole, itraconazole, fluconazole, terbinafin, naphthyfin, butenafin, amolorfin, lyranaphthate, naphthiomate N, tolnaphthate (naphthiomate T), tolciclate, undecylenic acid, phenyl-11-iodo-10-undecynoate, salicylic acid, siccanin, trichomycin, pyrrolnitrin, nystatin, pimalycin, griseofulvin, bariotin, amphotericin B, exalamide, cyclopyroxuoramine, haloprogin, zinc diethylthiocarbamate, thiantol, flucytosine, 2,4,6-tribromophenylcaproic acid ester, trimethylcetylammonium pentachlorophenate, sulfur, the bark of Rose of Sharon (Hibiscus syriacus Linne), and salts thereof.

Examples of central nervous system acting agent (hypnotic sedative agent, antiepileptic agent, and agent for mental nerves) include fluphenazine, thioridazine, diazepam, chlorpromazine, nitrazepam, estazolam, triazolam, nimethazepam, flunitrazepam, haloxazolam, flurazepam, clonazepam, propericyazine, prochlorperazine, alprazolam, oxazepam, oxazolam, cloxazolam, prazepam, flutazolam, mexazolam, lorazepam, fludiazepam, bromazepam, metazepam, and others.

Examples of diuretic agent include hydrothiazide, bendroflunathiazide, ethiazide, cyclopentiazide, hydrochlorothiazide, penflutide, methyclothiazide, furosemide, metolazone, polythiazide, bendroflumethiazide, and others.

Examples of hypotensive agent include clonizine, ulceroxylon, rescinnamine, dihydroergotoxin mesylate, reserpine, plazocin, captopril, pindolol, enalapril maleate, and others.

Examples of coronary vasodilator agent include nitroglycerin, nitroglycol, isosorbite dinitrate, papaverine hydrochloride, dipyridamole, efloxate, trimethazine, nicolanzyl, cinnarizine, nylidone, morcidomine, nifedipine, and others.

Examples of antitussive expectorant agent include codeine phosphate, dihydrocodeine phosphate, ephedrine hydrochloride, clorprenaline hydrochloride, fenoterol hydrobromide, salbutamol sulfate, dimemorphan phosphate, azelastine hydrochloride, clenbuterol hydrochloride, tulobuterol hydrochloride, trimethoxynol hydrochloride, procaterol hydrochloride, bromhexine hydrochloride, tolanilaste, tipepidine hidenzate, ketotiphen fumarate, phormoterol fumarate, benzproperine phosphate, glycyrrhizic acid, and others.

Examples of antihistamic agent include diphenhydramine hydrochloride, triprolidine hydrochloride, isothipendyl hydrochloride, promethazine hydrochloride, chlorpheniramine maleate, ciproheptazine hydrochloride, clemastine fumarate, carbinoxamine maleate, dimethindene maleate, and others.

Examples of antiarrhythmic agent include alprenotol, oxprenolol, bucumolol, bupranolol, pindolol, indenolol, calteolol, buphetolol, propranolol, timolol, and others.

Examples of cardiotonic agent include digitalis, ubidecalenon, digoxin, methyl digoxin, deslanoside, and others.

Examples of contraceptive drugs include estradiol enanthate, estradiol cypionate, levonorgestrel, estradiol, and others.

Examples of adrenocortical hormone agent include hydrocortisone acetate, hydrocortisone, prednisolone, triamcinolone acetonide, dexamethasone phosphate, methylprednisolone, dyclorisone acetate, methyprednisolone acetate, fluocinolone acetonide, dexamethasone acetate, dexamethasone, fluorometholone, betamethasone sodium phosphate, betamethasone, betamethasone valerate, beclomethasone propionate, fludroxycortide, hydrocortisone butyrate, betamethasone dipropionate, fluocinonide, clobetazole propionate, diflucortolone valerate, halcinonide, amcinonide, prednisolone valerate, and others.

Examples of local anesthetic agent include lidocaine, ethyl aminobenzoate, procaine hydrochloride, dibucaine, procaine, and others.

These pharmaceutically effective components are used either as one kind or by suitably blending two or more kinds.

The adhesive patch used in the present invention can also be used for an emergency adhesive plaster, a surgical dressing for the purpose of protecting the scar after an operation, a surgical drape for the purpose of preventing the contamination of the incised part with bacteria, a tape for reinforcing and fixing the incised and sutured part, and further as a taping used at the time of sports, and the like.

The backing used in the adhesive patch used in the present invention is selected, for example, from films or sheets of polyethylene, polypropylene, polybutadiene, ethylene-vinyl acetate copolymer, polyvinyl chloride, polyester (for example, polyethylene terephthalate (PET) or the like), nylon, and polyurethane, or porous articles or foamed articles thereof, and extendible or non-extendible backings such as paper, cloth, and non-woven cloth.

Next, as a method for producing an adhesive patch used in the present invention, one can perform according to a method that is conventionally carried out. By raising one example, one can produce the adhesive patch by adding and uniformly dispersing a sucrose stearic acid ester into a base component that has been dissolved or kneaded, further adding a pharmaceutically effective component or the like in accordance with the needs, and directly extending the mixture onto a tape or a sheet base material (backing), or extending the mixture onto paper, film, or the like that has once been subjected to a releasing treatment, and thereafter press-bonding and transcribing the mixture onto a base material (backing) to be used.

Regarding the adhesive patch used in the present invention obtained in the above-described manner, blending of a sucrose stearic acid ester is essential as will be described in the later-described Test Examples and Examples, and the sucrose stearic acid ester blended into the base restrains the physical actions such as generation of stress onto the skin at the time of application that causes a rash and tension of the skin and/or the hair at the time of peeling off. Therefore, it is surmised that the sucrose stearic acid ester blended into the base removes the cause of a rash due to the physical factors specific to adhesive patches. Also, the adhesiveness is retained well, and such functions and effects have not been obtained by water-soluble polymers, surfactants, fatty acid esters, polyhydric alcohols, and others that are blended in conventional adhesive patches.

The adhesive patch for external use according to the present invention will be an ideal, non-irritative to lowly-irritative adhesive patch for external use having properties that have been inconceivable in conventional adhesive patches for external use, in that "the physical actions such as the restraint of the stress onto the skin at the time of application and at the time of peeling off are removed while sufficiently retaining the adhesiveness to the skin".

Hereafter, functions and effects of the present invention will be described in further detail by way of Examples and Test Examples; however, the present invention is in no way limited by these Examples and Test Examples.

### Example 1

Into a base component made of 22 wt% of polyisoprene IR-2200 (manufactured by ZEON CORPORATION), 12 wt% of polyisobutylene L-100 (manufactured by Exxon Mobil Corporation), 8 wt% of styrene-isoprene-styrene block copolymer Quintac 3710 (manufactured by Shell Chemicals Japan Ltd.), 16 wt% of Alcon-P100 (manufactured by Arakawa Chemical Industries,Ltd.), and 16 wt% of liquid paraffin, 6 wt% of zinc oxide, 4 wt% of hydrous silica, and 1.0 wt% of sucrose fatty acid ester [Ryoutou Sugar Ester S-370F (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a staple fiber muslin base cloth so as to have a thickness of 200 µm, and the resultant was covered with a release paper that had been subjected to a silicone process. The resultant was cut into a desired shape to form an adhesive patch of the present invention.

### Example 2

Into a base component made of 20 wt% of polyisoprene IR-2200 (manufactured by ZEON CORPORATION), 12 wt% of polyisobutylene L-100 (manufactured by Exxon Mobil Corporation), 8 wt% of styrene-isoprene-styrene block copolymer Quintac 3710 (manufactured by Shell Chemicals Japan Ltd.), 16 wt% of Alcon-P100 (manufactured by Arakawa Chemical Industries,Ltd.), and 16 wt% of liquid paraffin, 6 wt% of zinc oxide, 4 wt% of hydrous silica, and 3.0 wt% of sucrose fatty acid ester [Ryoutou Sugar Ester S-370F (manufactured by Mitsubishi Chemical Foods)] were added and mixed. Subsequently, 6 wt% of methyl salicylate, 6 wt% of 1-menthol, 1 wt% of d1-camphor, and 2.0 wt% of tocopherol acetate were blended, and the mixture was extended on a staple fiber muslin base cloth so as to have a thickness of 200 µm, and the resultant was covered with a release paper that had been subjected to a silicone process. The resultant was cut into a desired shape to form an adhesive patch of the present invention.

### Example 3

Into a base component made of 7 wt% of polyisoprene IR-2200 (manufactured by ZEON CORPORATION), 13 wt% of polyisobutylene L-100 (manufactured by Exxon Mobil Corporation), 11 wt% of styrene-isoprene-styrene block copolymer Quintac 3710 (manufactured by Shell Chemicals Japan Ltd.), 19 wt% of Alcon-P100 (manufactured by Arakawa Chemical Industries,Ltd.), 5 wt% of polyisobutylene LM-MH (manufactured by Exxon Mobil Corporation), and 22 wt% of liquid paraffin, 4 wt% of zinc oxide, 3 wt% of hydrous silica, and 1.0 wt% of sucrose fatty acid ester [Surf Hope J1803F (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Example 4

Into a base component made of 7 wt% of polyisoprene IR-2200 (manufactured by ZEON CORPORATION), 13 wt% of polyisobutylene L-100 (manufactured by Exxon Mobil Corporation), 11 wt% of styrene-isoprene-styrene block copolymer Quintac 3710 (manufactured by Shell Chemicals Japan Ltd.), 18 wt% of Alcon-P100 (manufactured by Arakawa Chemical Industries,Ltd.), 5 wt% of polyisobutylene LM-MH (manufactured by Exxon Mobil Corporation), and 21 wt% of liquid paraffin, 4 wt% of zinc oxide, 3 wt% of hydrous silica, and 3.0 wt% of sucrose fatty acid ester [Surf Hope J1803F (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Example 5

Into a base component made of 7 wt% of polyisoprene IR-2200 (manufactured by ZEON CORPORATION), 13 wt% of polyisobutylene L-100 (manufactured by Exxon Mobil Corporation), 11 wt% of styrene-isoprene-styrene block copolymer Quintac 3710 (manufactured by Shell Chemicals Japan Ltd.), 19 wt% of Alcon-P100 (manufactured by Arakawa Chemical Industries,Ltd.), 5 wt% of polyisobutylene LM-MH (manufactured by Exxon Mobil Corporation), and 22 wt% of liquid paraffin, 4 wt% of zinc oxide, 3 wt% of hydrous silica, and 1.0 wt% of sucrose fatty acid ester [Surf Hope J1801 (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Example 6

Into a base component made of 7 wt% of polyisoprene IR-2200 (manufactured by ZEON CORPORATION), 13 wt% of polyisobutylene L-100 (manufactured by Exxon Mobil Corporation), 11 wt% of styrene-isoprene-styrene block copolymer Quintac 3710 (manufactured by Shell Chemicals Japan Ltd.), 19 wt% of Alcon-P100 (manufactured by Arakawa Chemical Industries,Ltd.), 5 wt% of polyisobutylene LM-MH (manufactured by Exxon Mobil Corporation), and 22 wt% of liquid paraffin, 4 wt% of zinc oxide, 3 wt% of hydrous silica, and 1.0 wt% of sucrose fatty acid ester [Surf Hope J1811 (manufactured by Mitsubishi Chemical Foods)] were added and mixed. Subsequently, 6 wt% of methyl salicylate, 5 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Example 7

Into a base component made of 6 wt% of polyisoprene IR-2200 (manufactured by ZEON CORPORATION), 13 wt% of polyisobutylene L-100 (manufactured by Exxon Mobil Corporation), 10 wt% of styrene-isoprene-styrene block copolymer Quintac 3710 (manufactured by Shell Chemicals Japan Ltd.), 18 wt% of Alcon-P100 (manufactured by Arakawa Chemical Industries,Ltd.), 7 wt% of polyisobutylene LM-MH (manufactured by Exxon Mobil Corporation), and 21 wt% of liquid paraffin, 4 wt% of zinc oxide, 3 wt% of hydrous silica, and 3.0 wt% of sucrose fatty acid ester [Surf Hope J1803F (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Example 8

Into a base component made of 7 wt% of polyisoprene IR-2200 (manufactured by ZEON CORPORATION), 13 wt% of polyisobutylene L-100 (manufactured by Exxon Mobil Corporation), 11 wt% of styrene-isoprene-styrene block copolymer Quintac 3710 (manufactured by Shell Chemicals Japan Ltd.), 19 wt% of Alcon-P100 (manufactured by Arakawa Chemical Industries,Ltd.), 5 wt% of polybutene 40-SH (manufactured by NOF CORPORATION), and 22 wt% of liquid paraffin, 4 wt% of zinc oxide, 3 wt% of hydrous silica, and 1.0 wt% of sucrose fatty acid ester [Surf Hope J1801 (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Example 9

Into a base component made of 7 wt% of polyisoprene IR-2200 (manufactured by ZEON CORPORATION), 13 wt% of polyisobutylene L-100 (manufactured by Exxon Mobil Corporation), 11 wt% of styrene-isoprene-styrene block copolymer Quintac 3710 (manufactured by Shell Chemicals Japan Ltd.), 19 wt% of YS Resin PX-1150N (manufactured by YASUHARA CHEMICAL Co., Ltd.), 5 wt% of polybutene 40-SH (manufactured by NOF CORPORATION), and 22 wt% of liquid paraffin, 4 wt% of zinc oxide, 3 wt% of hydrous silica, and 1.0 wt% of sucrose fatty acid ester [Surf Hope J1801 (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Example 10

Into a base component made of 7 wt% of polyisoprene IR-2200 (manufactured by ZEON CORPORATION), 13 wt% of polyisobutylene L-100 (manufactured by Exxon Mobil Corporation), 11 wt% of styrene-isoprene-styrene block copolymer Quintac 3710 (manufactured by Shell Chemicals Japan Ltd.), 19 wt% of Quinton B-170 (manufactured by ZEON CORPORATION), 5 wt% of polybutene 40-SH (manufactured by NOF CORPORATION), and 22 wt% of liquid paraffin, 4 wt% of zinc oxide, 3 wt% of hydrous silica, and 1.0 wt% of sucrose fatty acid ester [Surf Hope J1801 (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Example 11

Into a base component made of 7 wt% of polyisoprene IR-2200 (manufactured by ZEON CORPORATION), 14 wt% of polyisobutylene L-100 (manufactured by Exxon Mobil Corporation), 11 wt% of styrene-isoprene-styrene block copolymer Quintac 3710 (manufactured by Shell Chemicals Japan Ltd.), 20 wt% of Alcon-P100 (manufactured by Arakawa Chemical Industries,Ltd.), 2 wt% of polybutene 40-SH (manufactured by NOF CORPORATION), and 23 wt% of liquid paraffin, 4 wt% of zinc oxide, 3 wt% of hydrous silica, and 1.0 wt% of sucrose fatty acid ester [Surf Hope J1801 (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Example 12

Into a base component made of 7 wt% of polyisoprene IR-2200 (manufactured by ZEON CORPORATION), 14 wt% of polyisobutylene L-100 (manufactured by Exxon Mobil Corporation), 11 wt% of styrene-isoprene-styrene block copolymer Quintac 3710 (manufactured by Shell Chemicals Japan Ltd.), 20 wt% of YS Resin PX-1150N (manufactured by YASUHARA CHEMICAL Co., Ltd.), 2 wt% of polybutene 40-SH (manufactured by NOF CORPORATION), and 22 wt% of liquid paraffin, 4 wt% of zinc oxide, 3 wt% of hydrous silica, and 1.0 wt% of sucrose fatty acid ester [Surf Hope J1801 (manufactured by Mitsubishi Chemical Foods)] were added and mixed. Subsequently, 6 wt% of methyl salicylate, 6 wt% of 1-menthol, 1 wt% of dl-camphor, and 2.0 wt% of tocopherol acetate were blended, and the mixture was extended on a PET film so as to have a thickness of 200 µm, and the resultant was covered with a release paper that had been subjected to a silicone process. The resultant was cut into a desired shape to form an adhesive patch of the present invention.

### Comparative Example 1

An adhesive patch was prepared by removing the sucrose fatty acid ester from Example 1, thereby to form an adhesive patch of Comparative Example 1.

### Comparative Example 2

An adhesive patch was prepared by removing the sucrose fatty acid ester from Example 4, thereby to form an adhesive patch of Comparative Example 2.

### Comparative Example 3

An adhesive patch was prepared by removing the sucrose fatty acid ester from the adhesive patch of Example 7, thereby to form an adhesive patch of Comparative Example 3.

### Comparative Example 4

An adhesive patch was prepared by removing the sucrose fatty acid ester from Example 8, thereby to form an adhesive patch of Comparative Example 4.

### Comparative Example 5

An adhesive patch was prepared by removing the sucrose fatty acid ester from Example 11, thereby to form an adhesive patch of Comparative Example 5.

### Test Example 1. adhesiveness test

With regard to each of the adhesive patches of the Examples according to the present invention and each of the adhesive patches of the Comparative Examples, the probe tackiness value and the 180° peeling strength, which are considered to be physical indices of adhesiveness, were measured. There was no significant difference in the measured values, so that decrease in the measured values by addition of a sucrose fatty acid ester was not recognized with regard to the probe tackiness value and the 180° peeling strength.

### Test Example 2. function test

The adhesive patches of Examples 4, 5, 8, 9, and 10 according to the present invention and the adhesive patches of Comparative Examples 2, 4, and 5 were each applied for 6 hours on the outside of a forearm of 30 subjects, and a functional research was carried out by a questionnaire.

With the questionnaire, the declaration of rubor/rash after peeling off and the degree of adhesion were replied by numerical values that are ranked as follows. The result is shown in Table 1. Here, the adhesiveness index in Table 1 is an average value of the ranks.

### <adhesiveness index>

- 1.: peeling off of 1/2 or more
- 2.: peeling off of 1/3
- 3.: peeling off of 1/4
- 4.: peeling off of an end part
- 5.: no peeling off

**[Table 1]**

| | ADHESIVENESS INDEX |
|---|---|
| COMPARATIVE EXAMPLE 2 | 4.6 |
| EXAMPLE 4 | 4.25 |
| EXAMPLE 5 | 4.65 |
| COMPARATIVE EXAMPLE 4 | 4.50 |
| EXAMPLE 8 | 4.30 |
| EXAMPLE 9 | 4.65 |
| EXAMPLE 10 | 4.45 |
| COMPARATIVE EXAMPLE 5 | 4.25 |
| EXAMPLE 11 | 4.25 |
| EXAMPLE 12 | 4.55 |

As a result of the questionnaire, appearance of rubor/rash was recognized in the adhesive patches of Comparative Examples 2, 4, and 5; however, there was no appearance of rubor/rash in the adhesive patches of each of the Examples according to the present invention.

Also, as shown in Table 1, no decrease in the adhesive index was recognized. Further, since the average values of the adhesive indices are all 4 or above, an extremely good adhesiveness was shown such as "peeling off of an end part to no peeling off" in all cases with regard to the adhesiveness.

### Test Example 3. accumulated skin irritation evaluation on rabbits

The adhesive patches of Examples 1, 2, 8, 10, and 11 according to the present invention and the adhesive patches of Comparative Examples 1, 4, and 5 were applied and peeled off every one hour repeatedly for 8 times on the back of a rabbit from which hair had been removed. After 2 hours, after 24 hours, and after 48 hours, determination by eye inspection was carried out in accordance with the skin irritation property evaluation determination standard of Table 2. A skin irritation score at each time was calculated, and the irritation score at each time was converted into a percentage relative to the maximum skin irritation score, as an accumulated % skin irritation. A numerical value obtained by summing up (total value) was calculated as a comprehensive index of skin irritation. The result is shown in Table 3 and Fig. 1.

Here, the method of calculating the accumulated skin % irritation (2, 24, 48 hours and the total value) is as follows.

2-hour value: The irritation property at 2 hours after the peeling off of the specimen is determined by eye inspection according to an evaluation standard of erythema (0 to 3 points), edema (0 to 2 points), and incrustation (0 to 2 points), and each is scored. The sum of these will be a skin irritation score. Subsequently, this skin irritation score is divided by the maximum skin irritation score (3 + 2 + 2 = 7 points), and this is multiplied by 100. Namely, the ratio (%) of the skin irritation score after 2 hours relative to the maximum irritation score is calculated as a 2-hour value.

The 24-hour value and the 48-hour value are calculated in a similar manner.

The total value is calculated as a sum of the 2-hour, 24-hour, and 48-hour values.

**[Table 2]**

| degree of skin reaction | scores |
|---|---|
| erythema | |
| no erythema | 0 |
| slight erythema (degree of faint recognition) | 1 |
| definite erythema | 2 |
| erythema of middle degree to grave degree (deep scarlet color) | 3 |
| edema | |
| no edema | 0 |
| slight edema is recognized | 1 |
| definite edema is recognized | 2 |
| incrustation | |
| no incrustation | 0 |
| slight incrustation is recognized | 1 |
| definite incrustation is recognized | 2 |

**[Table 3]**

| | ACCUMULATED % SKIN IRRITATION |
|---|---|
| COMPARATIVE EXAMPLE 1 | 76 |
| EXAMPLE 1 | 19 |
| EXAMPLE 2 | 5 |
| COMPARATIVE EXAMPLE 4 | 117 |
| EXAMPLE 8 | 33 |
| EXAMPLE 10 | 83 |
| COMPARATIVE EXAMPLE 5 | 90 |
| EXAMPLE 11 | 52 |

As is clear from Table 3 and Fig. 1, in the case of an adhesive patch of each Example according to the present invention in which a sucrose fatty acid ester has been incorporated, the accumulated irritation of the skin is reduced to a large extent as compared with the case of an adhesive patch of each Comparative Example in which a sucrose fatty acid ester has not been incorporated.

### Industrial Applicability

As is clear from the result of the above-described Test Examples, the adhesive patch used in the present invention is an excellent adhesive patch for external use with reduced rash while retaining a good adhesiveness, and has an extremely wide range of use as an adhesive patch for various medical purposes.

## Claims

1. A sucrose stearic acid ester as a component in an adhesive patch for external use for use in the reduction of skin irritation and rash, wherein the adhesive patch is formed by incorporating the sucrose stearic acid ester into an adhesive base in an amount of 0.1 to 5.0 wt%.

2. The sucrose stearic acid ester for use according to claim 1, wherein the adhesive patch is formed by directly or indirectly extending and applying the adhesive base onto a backing.

3. The sucrose stearic acid ester for use according to any one of claims 1 to 2, wherein the adhesive base is a rubber base.

4. The sucrose stearic acid ester for use according to claim 3, wherein the rubber base is one or more kinds selected from styrene-isoprene-styrene copolymer, polyisobutylene, and polyisoprene.

5. The sucrose stearic acid ester for use according to claim 4, wherein the rubber base further contains polybutene.

6. The sucrose stearic acid ester for use according to any one of claims 1 to 2, wherein the adhesive base is a water-soluble polymer.

## Patentansprüche

1. Saccharosestearinsäureester als Komponente in einem Klebepflaster zur äußerlichen Verwendung zur Verwendung bei der Verringerung von Hautreizung und Rash, wobei das Klebepflaster durch Einarbeiten des Saccharosestearinsäureesters in eine Klebstoffgrundlage in einer Menge von 0,1 bis 5,0 Gew.-% gebildet wird.

2. Saccharosestearinsäureester zur Verwendung gemäß Anspruch 1, wobei das Klebepflaster durch direktes oder indirektes Verlängern und Auftragen der Klebstoffgrundlage auf einen Träger gebildet wird.

3. Saccharosestearinsäureester zur Verwendung gemäß einem der Ansprüche 1 bis 2, wobei die Klebstoffgrundlage eine Kautschukgrundlage ist.

4. Saccharosestearinsäureester zur Verwendung gemäß Anspruch 3, wobei die Kautschukgrundlage eine Art oder mehrere Arten, ausgewählt aus Styrol-Isopren-Styrol-Copolymer, Polyisobutylen und Polyisopren, ist.

5. Saccharosestearinsäureester zur Verwendung gemäß Anspruch 4, wobei die Kautschukgrundlage außerdem Polybuten enthält.

6. Saccharosestearinsäureester zur Verwendung gemäß einem der Ansprüche 1 bis 2, wobei die Klebstoffgrundlage ein wasserlösliches Polymer ist.

## Revendications

1. Ester d'acide stéarique de sucrose comme composant dans un timbre adhésif pour usage externe à utiliser pour réduire l'irritation et la rougeur de la peau, dans lequel le timbre adhésif est formé en incorporant l'ester d'acide stéarique de sucrose dans une base adhésive en une quantité comprise entre 0,1 et 5,0 % en poids.

2. Ester d'acide stéarique de sucrose à utiliser selon la revendication 1, dans lequel le timbre adhésif est formé en étendant et en appliquant directement ou indirectement la base adhésive sur un support.

3. Ester d'acide stéarique de sucrose à utiliser selon l'une quelconque des revendications 1 à 2, dans lequel la base adhésive est une base de caoutchouc.

4. Ester d'acide stéarique de sucrose à utiliser selon la revendication 3, dans lequel la base de caoutchouc est d'un ou de plusieurs type(s) sélectionné(s) parmi un copolymère de styrène-isoprène-styrène, le polyisobutylène et le polyisoprène.

5. Ester d'acide stéarique de sucrose à utiliser selon la revendication 4, dans lequel la base de caoutchouc contient en outre du polybutène.

6. Ester d'acide stéarique de sucrose à utiliser selon l'une quelconque des revendications 1 à 2, dans lequel la base adhésive est un polymère soluble dans l'eau.
